(19) Europäisches Patentamt
European Patent Office
Office européen des brevets

(11) Publication number: **0 261 958 A2**

# (12) EUROPEAN PATENT APPLICATION

(21) Application number: 87308436.2

(22) Date of filing: 23.09.87

(51) Int. Cl.[4]: C 10 L 1/24
C 10 L 1/14

(30) Priority: 24.09.86 GB 8622961
17.08.87 GB 8719423

(43) Date of publication of application:
30.03.88 Bulletin 88/13

(84) Designated Contracting States:
AT BE CH DE ES FR GB GR IT LI LU NL SE

(71) Applicant: EXXON CHEMICAL PATENTS INC.
1900 East Linden Avenue
Linden New Jersey 07036 (US)

(72) Inventor: Lewtas, Kenneth
21 Blackcroft
Wantage Oxfordshire OX12 9EX (GB)

Lehmann, Edwin William
6 Cottage Road Stanford-in-the-Vale
Faringdon Oxfordshire SN7 8HX (GB)

Kerwood, Richard Dix
17 Sovereign Close
Didcot Oxfordshire 0X11 8TR (GB)

Bartz, Kenneth William
1 Courtenay Close
Sutton Courtenay Oxfordshire (GB)

Bland, Jacqueline Dawn
"Materiana" Springfield Road
Wantage Oxfordshire 0X12 8HB (GB)

Gillingham, David Paul
4 Wakefield Close Freshbrook
Swindon§Wiltshire SN5 8Q (GB)

Maddox, John Edward
18 Woodchester-Westlea
Swindon Wiltshire SN5 7BQ (GB)

(74) Representative: Bawden, Peter Charles et al
EXXON CHEMICAL LIMITED EXXON CHEMICAL
TECHNOLOGY CENTRE PO Box 1
Abingdon Oxfordshire OX13 6BB (GB)

## (54) Middle distillate compositions with reduced wax crystal size.

(57) Distillate fuel oil boiling in the range 120°C to 500°C which has a wax content of at least 0.3 wt.% at a temperature of 10°C below the Wax Appearance Temperature, the wax crystals at that temperature having an average particle size less than 4000 nanometres preferably less than 1000 nanometres.

EP 0 261 958 A2

## Description

Middle Distillate Compositions with Reduced Wax Crystal Size

This invention relates to improved distillate fuel oil.

Mineral oils containing paraffin wax such as the distillate fuels used as diesel fuel and heating oil have the characteristic of becoming less fluid as the temperature of the oil decreases. This loss of fluidity is due to the crystallisation of the wax into plate-like crystals which eventually form a spongy mass entrapping the oil therein, the temperature at which the wax crystals begin to form being known as the Cloud Point, the temperature at which the wax prevents the oil pouring is known as the Pour Point.

It has long been known that various additives act as Pour Point depressants when blended with waxy mineral oils. These compositions modify the size and shape of wax crystals and reduce the cohesive forces between the crystals and between the wax and the oil in such a manner as to permit the oil to remain fluid at a lower temperature so being pourable and able to pass through coarse filters.

Various Pour Point depressants have been described in the literature and several of these are in commercial use. For example, US Patent No. 3,048,479 teaches the use of copolymers of ethylene and $C_1$-$C_5$- vinyl esters, e.g. vinyl acetate, as Pour Point depressants for fuels, specifically heating oils, diesel and jet fuels. Hydrocarbon polymeric pour depressants based on ethylene and higher alpha-olefins, e.g. propylene, are also known. US Patent 3,252,771 relates to the use of polymers of $C_{16}$ to $C_{18}$ alpha-olefins with aluminium trichloride/alkyl halide catalysts as pour depressants in distillate fuels of the "broad boiling", easy-to-treat types available in the United States in the early 1960's.

In the late 1960's, early 1970's, greater emphasis was placed upon improving the filterability of oils at temperatures between the Cloud Point and the Pour Point as determined by the more severe Cold Filter Plugging Point (CFPP) Test (IP 309/80) and many patents have since been issued relating to additives for improving fuel performance in this test. US Patent 3,961,916 teaches the use of a mixture of copolymers, to control the size of the wax crystals. United Kingdom Patent 1,263,152 suggests that the size of the wax crystals may be controlled by using a copolymer having a lower degree of side chain branching.

It has also been proposed in, for example, United Kingdom Patent 1,469,016, that the copolymers of di-n-alkyl fumarates and vinyl acetate previously used as pour depressants for lubricating oils may be used as co-additives with ethylene/vinyl acetate copolymers in the treatment of distillate fuels with high final boiling points to improve their low temperature flow properties.

It has also been proposed to use additives based on olefin/maleic anhydride copolymers. For example, U.S. Patent 2,542,542 uses copolymers of olefins such as octadecene with maleic anhydride esterified with an alcohol such as lauryl alcohol as pour depressants and United Kingdom Patent 1,468,588 uses copolymers of $C_{22}$-$C_{28}$ olefins with maleic anhydride esterified with behenyl alcohol as co-additives for distillate fuels. Similarly, Japanese Patent Publication 5,654,037 uses olefin/maleic anhydride copolymers which have been reacted with amines as Pour Point depressants.

Japanese Patent Publication 5,654,038 uses the derivatives of the olefin/maleic anhydride copolymers together with conventional middle distillate flow improvers such as ethylene vinyl acetate copolymers. Japanese Patent Publication 5,540,640 discloses the use of olefin/maleic anhydride copolymers (not esterified) and states that the olefins used should contain more than 20 carbon atoms to obtain CFPP activity. United Kingdom Patent 2,192,012 uses mixtures of certain esterified olefin/maleic anhydride copolymers and low molecular weight polythene, the esterified copolymers being ineffective when used as sole additives.

The improvement in CFPP activity from incorporation of the additives of these Patents is achieved by modifying the size and shape of the wax crystals forming to produce mostly needle like crystals generally of particle size 10000 nanometres or bigger typically 30000 to 100000 nanometres. In operation of diesel engines at low temperatures, these crystals do not pass through the vehicle paper fuel filters but form a permeable cake on the filter allowing the liquid fuel to pass, the wax crystals will subsequently dissolve as the engine and the fuel heats up, which can be by the bulk fuel being heated by recycled fuel. A build up of wax can, however, block the filters, leading to diesel vehicle starting problems and problems at the start of driving in cold weather similarly it can lead to failure of fuel heating systems.

We have now surprisingly found that waxy fuels having wax crystals of sufficiently small size at low temperatures to pass through paper main filters typically used in diesel engines may be obtained by the addition of certain additives.

The present invention therefore provides distillate fuel oil boiling in the range 120°C to 500°C which has a wax content of at least 0.5 wt.% at a temperature of 10°C below the Wax Appearance Temperature, the wax crystals at that temperature having an average particle size less than 4000 nanometres.

The Wax Appearance Temperature (WAT) of the fuel is measured by differential scanning calorimetry (DSC). In this test a small sample of fuel (25 microlitres) is cooled at 2°C/minute together with a reference sample of similar thermal capacity but which will not precipitate wax in the temperature range of interest (such as kerosene). An exotherm is observed when crystallisation commences in the sample. For example, the WAT of the fuel may be measured by the extrapolation technique on a Mettler TA 2000B Differential Scanning Calorimeter.

The wax content of the fuel is derived from the DSC trace by integrating the area enclosed by the base line and the exotherm down to the specified temperature. The calibration having been previously performed on a

known amount of crystallising wax.

The wax crystal average particle size is measured by analysing a Scanning Electron Micrography of a fuel sample at a magnification of between 4000 and 8000 and measuring the longest dimension of at least 40 out of 88 points on a predetermined grid. We find that providing the average size is less than 4000 nanometres the wax will begin to pass through the typical paper filters used in diesel engines together with the fuel although we prefer that the size be below 3000 nanometres, more preferably below 2000, even more preferably below 1500 nanometres most preferably below 1000 nanometres where the real benefits of passage of the crystals through the paper fuel filters is achieved. The actual size attainable depends upon the original nature of the fuel and the nature and amount of additive used but we have found that these sizes and smaller are attainable.

The ability to obtain such small wax crystals in the fuel results in significant benefit in diesel engine operability. This may be demonstrated by pumping stirred fuel through a diesel filter paper as used in a V.W. Golf or Cummins diesel engine at from 8 to 15 ml/second and 1.0 to 2.4 litres per minute per square metre of filter surface area at a temperature at least 5° C below the wax appearance temperature with at least 0.5 wt.% of the fuel being present in the form of solid wax. Both fuel and wax are considered to successfully pass through the filter if one or more of the following criteria are satisfied.

(i) When 18 to 20 litres of fuel have passed through the filter the pressure drop across the filter does not exceed 50 kiloPascals (kPa), preferably 25 kPa, more preferably 10 kPa, most preferably 5 kPa.

(ii) At least 60%, preferably at least 80%, more preferably at least 90 wt.% of the wax present in the original fuel, as determined by the DSC test previously described is found to be present in the fuel leaving the filter.

(iii) Whilst pumping 18-20 litres of fuel through the filter, the flow rate always remains at above 60% of the initial flow rate and preferably above 80%.

The portion of crystals passing through the vehicle filter and the operability benefit arising from small crystals are highly dependant on crystal length although crystal shape is also significant. We find that cube-like crystals tend to pass through filters slightly more easily than do flat crystals and, when they do not pass, offer less resistance to fuel flow. Nonetheless the preferred crystal form is a flat form, which in principle allows more tax to be laid down as the temperature falls, and therefore more wax precipitates before the critical crystal length is reached than would a crystal of the same length in cube-like form.

The fuels of the present invention have outstanding benefits compared to previous distillate fuels improved in their cold flow properties by the addition of conventional additives. The fuels also have improved cold start performance at low temperatures not relying on recirculation of warm fuel to remove undesirable wax deposits. Furthermore the wax crystals tend to remain in suspension rather than settle and form waxy layers in storage tanks as occurs with fuels treated with conventional additives giving benefits in distribution. In addition the fuels frequently have improved operability in the Cold Climate Chassis Dynamometer Test as compared with fuel containing conventional additives. In many instances the fuels also have improved CFPP performance.

Distillate fuels within the general class of those boiling from 120° C to 500° C vary significantly in their boiling characteristics, n-alkane distributions and wax contents. With fuels from Northern Europe generally having lower final boiling points and cloud points than those in Southern Europe. The wax contents are generally greater than 1.5% (at 10° C below the WAT). Similarly fuels from other countries round the world vary in the same way with their respective climates but the wax content also depends on the source of the crude oil. A fuel derived from a Middle Eastern crude is likely to have a lower wax content than one derived from one of the waxy crudes such as those from China and Australia.

The extent to which the very small crystals can be obtained depends upon the nature of the fuel itself, and in some fuels it may not be possible to produce the extremely small crystals required by the present invention. If this situation arises the fuel characteristics may however be modified to enable such small crystals to be obtained by for example adjustment of refinery conditions and blending to enable the use of suitable additives.

The additives we prefer to use are compounds of the general formula

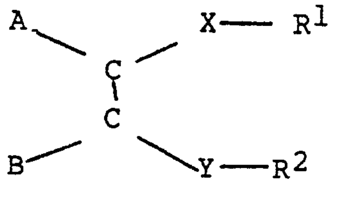

in whch -Y-$R^2$ is $SO_3^{(-)}{}^{(+)}NR^3_3R^2$, -$SO_3^{(-)}{}^{(+)}HNR^3_2R^2$, -$SO_3^{(-)}{}^{(+)}H_2NR^3R^2$, -$SO_3^{(-)}{}^{(+)}H_3NR^2$, -$SO_2NR^3R^2$ or -$SO_3R^2$;

-X-$R^1$ is -Y-$R^2$ or -$CONR^3R^1$, -$CO_2^{(-)}{}^{(+)}NR^3_3R^1$, -$CO_2^{(-)}{}^{(+)}HNR^3_2R^1$, -$CO_2^{(-)}{}^{(+)}H_2NR^3R^1$,-$CO_2^{(-)}{}^{(+)}H_3NR^1$, -$R^4$-$COOR_1$, -$NR^3COR^1$, $R^4OR^1$, -$R^4OCOR^1$,-$R^4R^1$, -N($COR^3$)$R^1$ or $Z^{(-)}{}^{(+)}NR^3_3R^1$;

-$Z^{(-)}$ is $SO_3^{(-)}$ or -$CO_2^{(-)}$;

$R^1$ and $R^2$ are alkyl, alkoxy alkyl or polyalkoxy alkyl containing at least 10 carbon atoms in the main chain; $R^3$ is hydrocarbyl and each $R^3$ may be the same or different and $R^4$ is nothing or is $C_1$ to $C_5$ alkylene and in

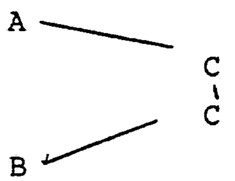

the carbon-carbon (C-C) bond is either a) ethylenically unsaturated when A and B may be alkyl, alkenyl or substituted hydrocarbyl groups or b) part of a cyclic structure which may be aromatic, polynuclear aromatic or cyclo-aliphatic, it is preferred that $X\text{-}R^1$ and $Y\text{-}R^2$ between them contain at least three alkyl, alkoxyalkyl or polyalkoxyalkyl groups.

The ring atoms in such cyclic compound are preferably carbon atoms, but could, however, include a ring N, S or O atom to give a heterocyclic compound.

Examples of aromatic based compound from which the additives may be prepared are

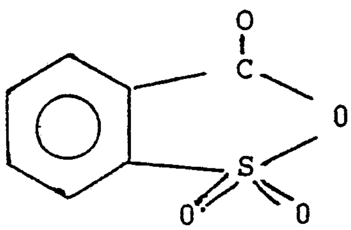

In which the aromatic group may be substituted;
Alternatively they may be obtained from polycyclic compounds, that is those having two or more ring structures which can take various forms. They can be (a) condensed benzene structures, (b) condensed ring structures where none or not all rings are benzene, (c) rings joined "end-on", (d) heterocyclic compounds (e) non-aromatic or partially saturated ring systems or (f) three-dimensional structures.

Condensed benzene structures from which the compounds may be derived include for example naphthalene, anthracene, phenathrene and pyrene.

The condensed ring structures where none or not all rings are benzene include for example Azulene, Indene, Hydroindene, Fluorene, Diphenylene. Compounds where rings are joined end-on include for example diphenyl. Suitable heterocyclic compounds from which they may be derived include for example Quinoline, Pyrindine, Indole, 2:3 dihydroindole, benzofuran, coumarin, isocoumarin, benzothiophen, carbazole and thiodiphenylamine. Suitable non-aromatic or partially saturated ring systems include decalin (decahydronaphthalene), α-Pinene, cadinene, bornylene. Suitable 3-dimensional compounds include for example norbornene, bicycloheptane (norbornane), bicyclo octane and bicyclo octene.

The two substituents X and Y must be attached to adjoining ring atoms in the ring when there is only one ring or to adjoining ring atoms in one of the rings where the compound is polycyclic. In the latter case this means that if one were to use naphthalene these substituents could not be attached to the 1,8- or 4,5-positions, but would have to be attached to the 1,2-, 2,3-, 3,4-, 5,6-, 6,7- or 7,8- positions.

These compounds are reacted to give the esters, amines, amides, half-esters/half amides, half ethers or salts used as the additives. Preferred additives are the salts of a secondary amine which has a hydrogen- and carbon-containing group or groups containing at least 10 preferably at least 12 carbon atoms. Such amines or salts may be prepared by reacting the acid or anhydride previously described with an amine or by reacting a secondary amine derivative with carboxylic acids or anhydrides. Removal of water and heating are generally necessary to prepare the amides from the acids. Alternatively the carboxylic acid may be reacted with an alcohol containing at least 10 carbon atoms or a mixture of an alcohol and an amine.

The hydrogen- and carbon-containing groups in the substituents are preferably hydrocarbyl groups, although halogenated hydrocarbyl groups could be used, preferably only containing a small proportion of halogen atoms (e.g. chlorine atoms), for example less than 20 weight per cent. The hydrocarbyl groups are preferably aliphatic, e.g. alkylene. They are preferably straight chain. Unsaturated hydrocarbyl groups, e.g. alkenyl, could be used but they are not preferred.

The alkyl groups preferably have at least 10 carbon atoms, preferably 12 to 22 carbon atoms, for example 14 to 20 carbon atoms and are preferably straight chain or branched at the 1 or 2 positions. If branching exists in over 20% of the alkyl chains then the branches must be methyl. The other hydrogen- and carbon-containing groups can be shorter e.g. less than 6 carbon atoms or may if desired have at least 10 carbon atoms. Suitable alkyl groups include methyl, ethyl, propyl, hexyl, decyl, dodecyl, tetradecyl, eicosyl and docosyl (behenyl). Suitable alkylene groups include hexylene, octylene, dodecylene and hexadecylene but these are not preferred.

In the preferred embodiment where the intermediate is reacted with the secondary amine, one of the substituents will preferably be an amide and the other will be an amine or dialkylammonium salt of the secondary amine. The especially preferred additives are the amides and amine salts of secondary amines.

In order to obtain the fuels of this invention these additives will generally be used in combination with other additives and Examples of the other additives include those termed as "comb" polymers which have the general formula:

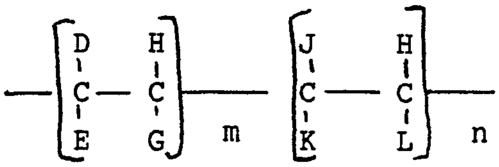

where D = R, CO.OR, OCO.R, R′CO.OR or OR
E = H or $CH_3$ or D or R′
G = H, or D
m = 1.0 (homopolymer) to 0.4 (mole ratio)
J = H, R′, Aryl or Heterocyclic group, R′CO.OR
K = H, CO.OR′, OCO.R′, OR′, $CO_2H$
L = H, R′, CO.OR′, OCO.R′, Aryl, $CO_2H$
n = 0.0 to 0.6 (mole ratio)
$R \geqq C_{10}$
$R \geqq C_1$

Another monomer may be terpolymerized if necessary

Where these other additives are copolymers of alpha olefins and maleic anhydrides they may conveniently be prepared by polymerizing the monomers solventless or in a solution of a hydrocarbon solvent such as heptane, benzene, cyclohexane, or white oil, at a temperature generally in the range of from 20° C to 150° C and usually promoted with a peroxide or azo type catalyst, such as benzoyl peroxide or azo-di-isobutyro-nitrile, under a blanket of an inert gas such as nitrogen or carbon dioxide, in order to exclude oxgen. It is preferred but not essential that equimolar amounts of the olefin and maleic anhydride be used although molar proportions in the range of 2 to 1 and 1 to 2 are suitable. Examples of olefins that may be copolymerized with maleic anhydride are 1-decene, 1-dodecene, 1-tetradecene, 1-hexadecene, 1-octodecene.

The copolymer of the olefin and maleic anhydride may be esterified by any suitable technique and although preferred it is not essential that the maleic anhydride be at least 50% esterified. Examples of alcohols which may be used include n-decan-1-ol, n-dodecan-1-ol, n-tetradecan-1-ol, n-hexadecan-1-ol, n-octadecan-1-ol. The alcohols may also include up to one methyl branch per chain, for example, 1-methyl, pentadecan-1-ol, 2-methyl, tridecan-1-ol. The alcohol may be a mixture of normal and single methyl branched alcohols. Each alcohol may be used to esterify copolymers of maleic anhydride with any of the olefins. It is preferred to use pure alcohols rather than the commercially available alcohol mixtures but if mixtures are used then $R^1$ refers to the average number of carbon atoms in the alkyl group, if alcohols the contain a branch at the 1 or 2 positions are used $R^1$ refers to the straight chain backbone segment of the alcohol. When mixtures are used, it is important that no more than 15% of the $R^1$ groups have the value $R^1+2$. The choice of the alcohol will, of course, depend upon the choice of the olefin copolymerised with maleic anhydride so that $R + R^1$ is within the range 18 to 38. The preferred value of $R + R^1$ may depend upon the boiling characteristics of the fuel in which the additive is to be used.

These comb polymers may also be fumarate polymers and copolymers such as those described in our European Patent Applications 0153176, 0153177, 85301047 and 85301048. Other suitable comb polymers are the polymers and copolymers of alpha olefins and the esterified copolymers of styrene and maleic anhydride.

Examples of other additives which may be used together with the cyclic compound are the polyoxyalkylene esters, ethers, ester/ethers and mixtures thereof, particularly those containing at least one, preferably at least two $C_{10}$ to $C_{30}$ linear saturated alkyl groups and a polyoxyalkylene glycol grup of molecular weight 100 to 5,000 preferably 200 to 5,000, the alkyl group in said polyoxyalkylene glycol containing from 1 to 4 carbon atoms. These materials form the subject of European Patent 0,061,895 B. Other such additives are described in United States Patent 4 491 455.

The preferred esters, ethers or ester/ethers useful in the present invention may be structurally depicted by the formula:

R-O(A)-O-R″

where R and R″ are the same or different and may be i) n-alkyl

ii)

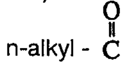

iii)

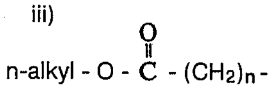

iv)

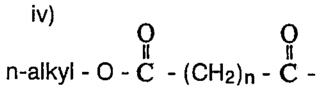

the alkyl group being linear and saturated and containing 10 to 30 carbon atoms, and A represents the polyoxyalkylene segment of the glycol in which the alkylene group has 1 to 4 carbon atoms, such as polyoxymethylene, polyoxyethylene or polyoxytrimethylene moiety which is substantially linear; some degree of branching with lower alkyl side chains (such as in polyoxypropylene glycol) may be tolerated but it is preferred the glycol should be substantially linear, A may also contain nitrogen.

Suitable glycols generally are the suitable linear polyethylene glycols (PEG) and polypropylene glycols (PPG) having a molecular weight of about 100 to 5,000, preferably about 200 to 2,000. Esters are preferred and fatty acids containing from 10-30 carbon atoms are useful for reacting with the glycols to form the ester additives and it is preferred to use a $C_{18}$-$C_{24}$ fatty acid, especially behenic acids. The esters may also be prepared by esterifying polyethoxylated fatty acids or polyethoxylated alcohols.

Polyoxyalkylene diesters, diethers, ether/esters and mixtures thereof are suitable as additives with diesters preferred for use in narrow boiling distillates whilst minor amounts of monoethers and monoesters may also be present and are often formed in the manufacturing process. It is important for additive performance that a major amount of the dialkyl compound is present. In particular, stearic or behenic diesters or polyethyleneglycol, polypropylene glycol or polyethylene /polyproylene glycol mixtures are preferred.

The additives used may also contain ethylene unsaturated ester copolymer flow improvers. The unsaturated monomers which may be copolymerised with ethylene include unsaturated mono and diesters of the general formula:

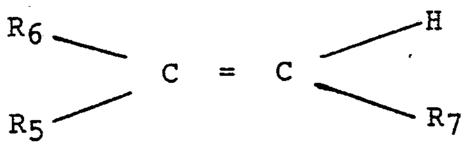

wherein $R_6$ is hydrogen or methyl, $R_5$ is a $-OOCR_8$ group wherein $R_8$ is hydrogen or a $C_1$ to $C_{28}$, more usually $C_1$ to $C_{17}$, and preferably a $C_1$ to $C_8$, straight or branched chain alkyl group; or $R_5$ is a $-COOR_8$ group wherein $R_8$ is as previously described but is not hydrogen and $R_7$ is hydrogen or $-COOR_8$ as previously defined. The monomer, when R6 and $R_7$ are hydrogen and R5 is $-OOCR_8$, includes vinyl alcohol esters of $C_1$ to $C_{29}$, more usually $C_1$ to $C_{18}$, monocarboxylic acid, and preferably $C_2$ to $C_{29}$, more usually $C_1$ to $C_{18}$, monocarboxylic acid, and preferably $C_2$ to $C_5$ monocarboxylic acid. Examples of vinyl esters which may be copolymerized with ethylene include vinyl acetate, vinyl propionate and vinyl butyrate or isobutyrate, vinyl acetate being preferred. When these are used, we prefer that the copolymers contain from 5 to 40 wt.% of the vinyl ester, more preferably from 10 to 35 wt.% vinyl ester. They may also be mixtures of the copolymers such as those described in US Patent 3,961,916. It is preferred that these copolymers have a number average molecular weight as measured by vapour phase osmometry of 1,000 to 10,000, preferably 1,000 to 5,000.

The additives used may also contain other polar compounds, either ionic or non-ionic, which have the capability in fuels of acting as wax crystal growth inhibitors. Polar nitrogen containing compounds have been found to be especially effective when used in combination with the glycol esters, ethers or ester/ethers. These polar compounds are generally amine salts and/or amides formed by reaction of at least one molar proportion of hydrocarbyl substituted amines with a molar proportion of hydrocarbyl acid having 1 to 4 carboxylic acid groups or their anhydrides; ester/amides may also be used containing 30 to 300, preferably 50 to 150 total carbon atoms. These nitrogen compounds are described in US Patent 4,211,534. Suitable amines are usually long chain $C_{12}$-$C_{40}$ primary, secondary, tertiary or quaternary amines or mixtures thereof but shorter chain amines may be used provided the resulting nitrogen compound is oil soluble and therefore normally containing about 30 to 300 total carbon atoms. The nitrogen compound preferably contains at least one straight chain $C_8$ to $C_{24}$ alkyl segment.

Suitable amines include primary, secondary, tertiary or quaternary, but preferably are secondary. Tertiary and quaternary amines can only form amine salts. Examples of amines include tetradecyl amine, cocoamine, hydrogenated tallow amine and the like. Examples of secondary amines include dioctacedyl amine, methyl-behenyl amine and the like. Amine mixtures are also suitable and many amines derived from natural materials are mixtures. The preferred amine is a secondary hydrogenated tallow amine of the formula $HNR_1R_2$ where in $R_1$ and $R_2$ are alkyl groups derived from hydrogenated tallow fat composed of approximately 4% $C_{14}$, 31% $C_{16}$, 59% $C_{18}$.

Examples of suitable carboxylic acids (and their anhydrides) for preparing these nitrogen compounds include cyclohexane, 1,2 dicarboxylic acid, cyclohexane 1,2 dicarboxylic acid, cyclopentane 1,2 dicarboxylic acid, naphthalene dicarboxylic acid and the like. Generally, these acids will have about 5-13 cabon atoms in the cyclic moiety. Preferred acids useful in the present invention are benzene dicarboxylic acids such as phthalic acid, isophthalic acid, and terephthalic acid. Phthalic acid or its anhydride is particularly preferred. The particularly preferred compound is the amide-amine salt formed by reacting 1 molar portion of phthalic anhydride with 2 molar portions of di-hydrogenated tallow amine. Another preferred compound is the diamide formed by dehydrating this amine-amine salt.

Hydrocarbon polymers may also be used as part of the additive combination to produce the fuels of the invention. These may be represented with the following general formula:

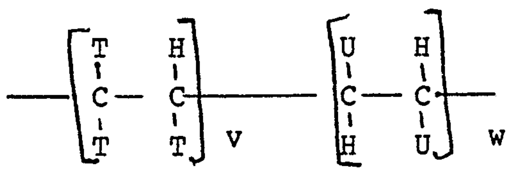

where T = H or R′
U = H, T or Aryl
v = 1.0 to 0.0 (mole ratio)
w = 0.0 to 1.0 (mole ratio)
$R^1$ is a normal alkyl group containing more than 10 carbon atoms.

These polymers may be made directly from ethylenically unsaturated monomers or indirectly by for example hydrogenating the polymer made from other monomers such as isoprene and butadiene.

A particularly preferred hydrocarbon polymer is a copolymer of ethylene and propylene having an ethylene content is preferably between 50 and 60% (w/w).

The amount of additive required to produce the distillate fuel oil of this invention will vary according to the fuel but is generally 0.001 to 0.5 wt.%, for example 0.01 to 0.1 wt.% (active matter) based on the weight of fuel. The additive may conveniently be dissolved in a suitable solvent to form a concentrate of from 20 to 90, e.g. 30 to 80 wt.% in the solvent. Suitable solvents include kerosene, aromatic naphthas, mineral lubricating oils etc.

The present invention is illustrated by the following examples in which the size of the wax crystals in the fuel was measured by placing samples of fuel in 2 oz. bottles in cold boxes held about 8°C above fuel cloud point for 1 hour while fuel temperature stabilises. The box is then cooled at 1°C an hour down to the test temperature, which is then held.

A pre-prepared filter carrier, consisting of a 10 mm diameter sintered ring, surrounded with a 1 mm wide annular metal ring, supporting a 200 nanometres rated silver membrane filter which is held in position by two vertical pins, is then placed on a vacuum unit. A vacuum of at least 80 kPa is applied, and the cooled fuel dripped onto the membrane from a clean dropping pipette until a small domed puddle just covers the membrane. The fuel is dropped slowly to sustain the puddle; after about 10-20 drops of fuel have been applied the puddle is allowed to draindown leaving a thin dull matt layer of fuel wet cake on the membrane. A thick layer of wax will not wash acceptably, and a thin one may be washed away. The optimal layer thickness is a function of crystal shape, with "leafy" crystals needing thinner layers than "nodular" crystals. It is important that the final cake have a matt appearance. A "shiny" cake indicates excessive residual fuel and crystal "smearing" and should be discarded.

The cake is then washed with a few drops of methyl ethyl ketone which are allowed to completely drain away. The process is repeated a number of times. When washing is complete the methyl ethyl ketone will disappear very quickly, leaving a "brilliant matt white" surface which will turn grey on application of another drop of methyl ethyl ketone.

The washed sample is then placed in a cold desiccator, and kept until ready for coating in the SEM. It may be necessary to keep the sample refrigerated to preserve the wax, in which case it should be stored in a cold box prior to transfer (in a suitable sample transfer container) to the SEM to avoid ice crystal formation on the sample surface.

During coating, the sample must be kept as cold as possible to minimise damage to the crystals. Electrical contact with the stage is best provided for by a retaining screw pressing the annular ring against the side of a well in the stage designed to permit the sample surface to lie on the instrument focal plane. Electrically conductive paint can also be used.

Once coated, the micrographs are obtained in a conventional way on the Scanning Electron Microscope. The photomicrographs are analysed to determine the average crystal size by fastening a transparent sheet with 88 points marked (as dots) at the intersections of a regular, evenly spaced grid 8 rows and 11 columns in size, to a suitable micrograph. The magnification should be such that only a few of the largest crystal are touched by more than one dot and a magnification of 4000 to 8000 times has proved suitable. At each grid point, if the dot touches a crystal dimension whose shape can be clearly defined, the crystal may be measured. A measure of "scatter", in the form of the Gaussian standard deviation of crystal length with Bessel correction applied is also taken.

The wax content before and after the filter is measured using a Differential Scanning Calorimeter DSC (such as the du Pont 9900 series) capable of generating a plot with an area of about 100 $cm^2$/1% of fuel in the form of wax with an instrument noise-induced output variation with a standard deviation less than 2% of the mean output signal.

The DSC is callibrated by using an additive to produce large crystals certain to be removed by the filter, running this calibration fuel at test temperature in the rig and measuring the WAT of the thus dewaxed fuel on the DSC. The samples of tank fuel and post-filter fuel to be tested are then analysed on the DSC, and for each fuel the area above base line down to the calibration fuel WAT determined.

The ratio of

$$\text{The ratio of } \frac{\text{DSC area for post filter sample}}{\text{DSC area for tank sample}} \times 100\%$$

is the % wax left after the filter.

The cloud point of distillate fuels was determined by the standard Cloud Point Test (IP-219 or ASTM-D 2500) other measures of the onset of crystallisation are the Wax Appearance Point (WAP) Test (ASTM D.3117-72) and the Wax Appearance Temperature (WAT) are measured by different scanning calorimetry using a Mettler TA 2000B differential scanning calorimeter.

The ability of the fuel to pass through a diesel vehicle main filter was determined in an apparatus consisting of a typical diesel vehicle main filter mounted in a standard housing in a fuel line; the Bosch Type as used in a 1980 VW Golf diesel passanger car, and a Cummins FF105 as used in the Cummins NTC engine series are appropriate. A reservoir and feed system capable of supplying half a normal fuel tank of fuel linked to a fuel injection pump as used in the VW Golf is used to draw fuel through the filter from the tank at constant flowrate, as in the vehicle. Instruments are provided to measure pressure drop across the filter, the flow rate from the injection pump and the unit temeratures. Receptacles are provided to receive the pumped fuel, both "injected" fuel and the surplus fuel.

In the test the tank is filled with 19 kilogrammes of fuel and leak tested. When satisfactory, the temperature is stabilised at an air temperature 8°C above fuel cloud point. The unit is then cooled at 3°C/hour to the desired test temperature, and held for at least 3 hours for fuel temperature to stabilise. The tank is vigorously shaken to fully disperse the wax present; a sample is taken from the tank and 1 litre of fuel removed through a sample point on the discharge line immediately after the tank and returned to the tank. The pump is then started, with pump rpm set to equate to pump rpm at a 110 kph road speed. In the case of the VW Golf this is 1900 rpm, corresponding to an engine speed of 3800 rpm. Pressure drop across the filter and flow rate of fuel from the injection pump are monitored until fuel is exhausted, typically 30 to 35 minutes.

If fuel feed to the injectors can be held at 2 ml/sec (surplus fuel will be about 6.5 - 7 ml/sec) the result is a "PASS". A drop in feed fuel flow to the injectors signifies a "BORDERLINE" result; zero flow a "FAIL".

Typically, a "PASS" result may be associated with an increasing pressure drop across the filter, which may rise as high as 60 kPa. Generally considerable proportions of wax must pass the filter for such a result to be achieved. A "GOOD PASS" is characterised by a run where the pressure drop across the filter does not rise about 10 kPa, and is the first indication that most of the wax has passed through the filter, an excellent result has a pressure drop below 5kPa.

Additionally, fuel samples are taken from "surplus" fuel and "injector feed" fuel, ideally every four minutes throughout the test. These samples, together with the pre-test tank samples, are compared by DSC to establish the proportion of feed wax that has passed through the filter. Samples of the pre-test fuel are also taken and SEM samples prepared from them after the test to compare wax crystal size and type with actual performance.

The additives used were

Additive 1

The N,N-dialkyl ammonium salt of 2-dialkylamido benzene sulphonate where the alkyl groups are $nC_{16-18}H_{33-37}$ prepared by reacting 1 mole of ortho-sulphobenzoic acid cyclic anhydride with 2 moles of di-(hydrogenated) tallow amine in a xylene solvent at 50% (w/w) concentration. The reaction mixture was stirred at between 100°C and the refluxing temperature. The solvent and chemicals should be kept as dry as possible so as not to enable hydrolysis of the anhydride.

The produce was analysed by 500 MHz Nuclear Magnetic Resonance Spectroscopy which confirmed the structure to be

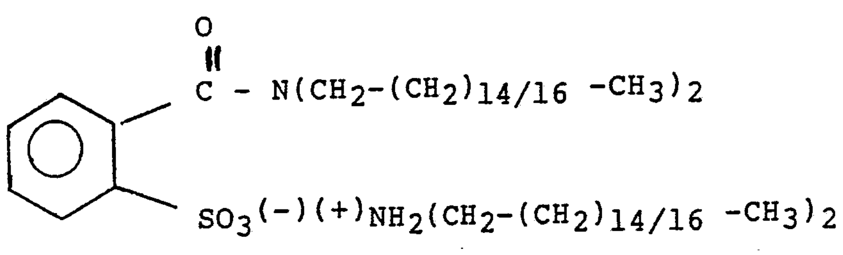

Additive 2

A copolymer of ethylene and vinyl acetate content 17 wt.% molecular weight 3500 and a degree of side chain branching of 8 methyls per 100 methylene groups as measured by 500 MHz NMR.

Additive 3

A styrene-dialkyl maleate copolymer made by esterifying a 1:1 molar styrene-maleic anhydride copolymer with 2 moles of a 1:1 molar mixture of $C_{12}H_{25}OH$ and $C_{14}H_{29}OH$ per mole of anhydride groups were used in the esterification (slight excess, 5% alcohol used) step using p-toluene sulphonic acid as the catalyst (1/10 mole) in xylene solvent, which gave a molecular weight (Mn) of 50,000 and contained 3% (w/w) unreacted alochol.

Additive 4

The dialkyl-ammonium salts of 2-N,N dialkylamido benzoate formed by mixing one molar proportion of phthalic anhydride with two molar proportions of di-hydrogenated tallow amine at 60°C.

The results were as follows.

Example 1

Fuel characteristics

Cloud point -14°C

Wax Appearance Temperature -18.6°C

Initial Boiling Point 178°C

20% 230°C

90% 318°C

Final Boiling Point 355°C

Wax content at -25°C 1.1 wt.%

250 p.p.m. of each of Additives 1, 2 and 3 were incorporated in the fuel and the test temperature was -25°C. The wax crystal size was found to be 1200 nanometres long and above 90 wt.% of the wax passed through the Cummins FF105 filter.

During the test, passage of wax was further evidenced by observing the pressure drop over the filter, which only increased by 2.2 kPa.

Example 2

Example 1 was repeated and the wax crystal size was found to be 1300 nanometres and the maximum final pressure drop across the filter was 3.4 kPa.

Example 3

Fuel characteristics

Cloud point 0°C

Wax Appearance Temperature -2.5°C

Initial Boiling Point 182°C

20% 220°C

90% 354°C

Final Boiling Point 385°C

Wax content at test temperature 1.6 wt%.

250 p.p.m. of each of Additives 1, 2 and 3 were used and the wax crystal size was found to be 1500 nanometres and about 75 wt% of the wax passed through the Bosch 145434106 filter at the test temperature of -8.5°C. The maximum pressure drop across the filter was 6.5 kPa.

Example 4

Example 3 was repeated and found to give wax crystal size 2000 nanometres long of which about 50 wt.% passed through filter giving a maximum pressure drop of 35.3 kPa.

Example 5

The fuel used in Example 3 was tested with 400 rpm of Additive 1 and 100 rpm of a mixture of Additive 2 and tested as in Example 3 at -8°C at which temperature the wax content 1.45 wt.%. The wax crystal size was found to be 2500 nanometres and 50 wt.% of the wax passed through the filter with a maximum final pressure drop of 67.1 kPa.

Comparative Example 6

The fuel used in Example 3 was treated with 500 ppm of a mixture of 4 parts of Additive 4 and 1 part of Additive 2 and tested at -8°C, the wax crystal size was found to be 6300 nanometres and 13 wt.% of the wax passed through the filter.

This example is among the very best examples of the prior art, where there is no crystal passage.

A scanning electron micrograph of the wax crystals forming in the fuel of Examples 1 to 6 are Figures 1 to 6 hereof.

Examples 1-4, therefore show that if crystals can pass through the filter reliably and the excellent cold temperature performance can be extended to higher fuel wax contents than heretofore practicable and also at temperatures further below fuel Wax Appearance Point than heretofore practicable. This is without regard to

fuel system consideration such as the ability of recycle fuel from the engine to warm the feed fuel being drawn from the fuel tank, the ratio of feed fuel flow to recycle fuel, the ratio of main filter surface area to feed fuel flow and the size and position of prefilters and screens.

Examples 1 to 3 show that for the filters tested crystal lengths below about 1800 nanometers result in dramatically better fuel performance.

Example 7

In this Example Additive 1 was added to a distillate fuel having the following characteristics:

IBP 180°C
20% 223°C
90% 336°C
FBP 365°C
Wax Appearance Temperature (WAT) - 5.5°C
Cloud Point - 3.5°C

For comparison purposes the following additives were also added to the distillate fuel:

Additive A: A mixture of ethylene/vinyl acetate copolymers, one of which was Additive 2 (1 part by weight) and the other one (3 parts by weight) with a vinyl acetate content of 36 wt.%, molecular weight (Mn) of 2000 a degree of side chain branching of between 2 and 3 methyls per 100 methylene groups as measured by 500 MHz NMR.

Additive B: A mixture of Additives 4 and 2 the mole ratio being 4:1.

Additive C: The dibehenate of a polyethylene glycol mixture having an average molecular weight of 600.

Additive D: An ethylene/propylene copolymer, the ethylene content being 56 wt.%, and the number average molecular weight being approximately 60,000.

The additive were added in the quantities shown in the following table and tests carried out according to the PCT, details of which are as follows:

PROGRAMMED COOLING TEST (PCT)

This is a slow cooling test designed to correlate with the pumping of a stored heating oil. The cold flow properties of the fuel containing the additives are determined by the PCT as follows. 300 ml of fuel are cooled linearly at 1°C/hour to the test temperature and the temperature then held constant. After 2 hours at the test temperature, approximately 20 ml of the surface layer is removed by suction to prevent the test being influenced by the abnormally large wax crystals which tend to form on the oil/air interface during cooling. Wax which has settled in the bottle is dispersed by gentle stirring, then a CFPPT(1) filter assembly is inserted. The tap is opened to apply a vacuum of 500 mm of mercury, and closed when 200 ml of fuel have passed through the filter into the graduated receiver: A PASS is recorded if the 200 ml are collected within tend second through a given mesh size or a FAIL if the flow rate is too slow indicating that the filter has become blocked.

(1) CFPPT - Cold Filter Plugging Point Test (CFPPT) described in detail in "Journal of the Institute of Petroleum", Vol. 52, No. 510, June 1966, pp. 173-185.

The mesh number passed at the test temperature is recorded.

TABLE

| Additive (mole ratio) | | Finest PCT mesh passed at -11°C 150 ppm | 250 ppm (active ingredient) |
|---|---|---|---|
| A | | 100 | 200 |
| 4 | | 60 | 120 |
| 1 | | 200 | 350 |
| 4/2 | (4/1) | 100 | 350 |
| 1/2 | (4/1) | 350 | 350 |
| 4/C | (9/1) | 150 | 250 |
| 1/C | (9/1) | 250 | 350 |
| 4/D | (9/1) | 150 | 350 |
| 1/D | (9/1) | 350 | 350 |

Thus, it can be seen that when only one additive is added, best results are achieved by additive 1, and when pairs of additives are used, best results are obtained when additive 1 is one of the pair.

Example 8

The fuel used in this Example had the following characteristics:
(ASTM-D86)
IBP 190°C
20% 246°C
90% 346°C
FBP 374°C
Wax Appearance Temperature -1.5°C
Cloud Point +2.0°C

It was treated with 1000 parts per million of active ingredient of the following additives:

(E) A mixture of Additive 2 (1 part by weight) and Additive 4 (9 parts by weight).

(F) The commercial ethylene vinyl acetate copolymer additive marketed by Exxon Chemicals as ECA 5920.

(G) A mixture of:
1 part Additive 1
1 part Additive 3
1 part Additive D
1 part of Additive K

(H) The commercial ethylene vinyl acetate copolymer additive marketed by Amoco as 2042E.

(I) The commercial ethylene vinyl propionate copolymer additive marketed by BASF as Keroflux 5486.

(J) No additive.

(K) The reaction product of 4 moles of di-hydrogenated-tallow amine and 1 mole of pyromellitic anhydride. The reaction is performed solventless at 150°C, stirring under nitrogen for 6 hours.

The following performance characteristics of these fuels were then measured.

(i) The ability of the fuel to pass through the diesel fuel main filter at -90°C, and the percentage of wax passing through the filter with the following results:

| Additive | Time to Failure | % of Wax Passing |
|---|---|---|
| E | 11 minutes | 18-30% |
| F | 16 minutes | 30% |
| G | No Failure | 90-100% |
| H | 15 minutes | 25% |
| I | 12 minutes | 25% |
| J | 9 minutes | 10% |

(ii) The pressure drop across the main filter against time and the results are shown graphically in Figure 7.

(iii) The wax settling in the fuels was measured by cooling fuel in a graduated measuring cylinder which contains 100 mls and this top level corresponds to 100% of the fuel's height. The cylinder is cooled at 1°C/hour from a temperature preferably 10°C above the fuel's Cloud Point but not less than 5°C above the fuel's Cloud Point to the test temperature, which is then held for a prescribed time. The test temperature and soak time depend upon the application, i.e. diesel fuel and heating oil. The test temperature is generally at least 5°C below the Cloud Point and the minimum cold soak time at the test temperature be at least 4 hours. Preferably the test temperature should be 10°C or more below the fuel's Cloud Point and the soak period should be 24 hours or more.

After the end of the soak period the measuring cylinder is examined and the extent of wax crystal settling is visually measured as the height of any wax longer above the bottom of the cylinder (0 mls) and expressed in terms of a percentage of the total volume (100 mls). Clear fuel may be seen above the settled wax crystals and this form of measurement is often sufficient to form a judgement on the wax settling. Often the fuel is cloudy above a settling wax crystal layer or the wax crystals can be seen to be visibly denser as they approach the bottom of the cylinder. In this case a more quantitative method of analysis is used. Here the top 5% (5 mls) of fuel is sucked off carefully and stored, the next 45% is sucked off and discarded, the next 5% is sucked off and stored, the next 35% is sucked off and discarded and finally the bottom 10% is collected after warming to dissolve the wax crystals. These stored samples will henceforth be referred to as Top, Middle and Bottom samples respectively. It is important that the vacuum applied be fairly low, i.e. 200 mm water pressure, and that the top of the pipette is placed just on the surface of the fuel to avoid currents in the liquid which could disturb the concentration of wax at different layers within the cylinder. The samples are then warmed to 60°C for 15 minutes and examined by Differential Scanning Calorimetry (DSC) as described elsewhere in this text.

The DSC technique involves using a machine such as the Dupont 9900 series or the Mettler TA 2000B. The latter machine was used here. A 25 ul sample is placed in the sample cell and regular kerosine in the reference cell then they are cooled at 22°C/minute from 60°C to at least 10°C but preferably 20°C above the Wax Appearance Temperature (WAT) then it is cooled at 2°C/minute to approximately 20°C below the WAT. A reference must be run of the unsettled, uncooled treated fuel. The extent of wax settling then correlates with the WAT (or WAT = WAT settled sample - WAT original). Negative values indicate dewaxing of the fuel and positive values indicate wax enrichment through settling. The wax content may also be used as a measure of settling from these samples. This is illustrated by % WAX or Δ% WAX (Δ%Wax = % Wax settled sample - % wax original) and, once again negative values indicated dewaxing of the fuel and positive values indicate wax enrichment through settling. These wax contents are derived by measuring the area under the DSC curve down to a specified temperature.

The fuel wax cooled at 1°C/hr from +10°C down to -9°C and cold soaked for 48 hours prior to testing. The results were as follows:

| Additive | Visual Wax Settling | WAT °C Data Top 5% | Middle 5% | Bottom 10% |
|---|---|---|---|---|
| E | Cloudy throughout Denser at bottom | -10.80 | -4.00 | -3.15 |
| F | 50% clear above | -13.35 | -0.80 | -0.40 |
| G | 100% | -7.85 | -7.40 | -7.50 |
| H | 35% clear above | -13.05 | -8.50 | +0.50 |
| I | 65% clear above | | | |
| J | 100% Semi-gel | -6.20 | -6.25 | -6.40 |

(The results are also shown graphically in Figure 8).

| WAT original (Unsettled Fuel) | | WAT(°C) (Settled Samples) Top 5% | Middle 5% | Bottom 10% |
|---|---|---|---|---|
| E | -6.00 | -4.80 | +2.00 | +2.85 |
| F | -5.15 | -8.20 | +4.35 | +4.75 |
| G | -7.75 | -0.10 | +0.35 | +0.25 |
| H | -5.00 | -8.05 | -3.50 | +4.50 |
| J | -6.20 | 0.00 | -0.05 | -0.20 |

(Note that significant depression of the WAT can be achieved by the most effective additive (G))

| | % WAX (Settled Samples) Top 5% | Middle 5% | Bottom 10% |
|---|---|---|---|
| E | -0.7 | +0.8 | +0.9 |
| F | -0.8 | +2.1 | +2.2 |
| H | -1.3 | -0.2 | +1.1 |
| G | ±0.0 | +0.3 | +0.1 |
| J | -0.1 | ±0.0 | ±0.1 |

(Note % WAX is measured by extending the original baseline and measuring the area from the WAT to -25°C here. A calibration having been previously performed on a known amount of crystallizing wax).

These results show that as the crystal size is reduced by the presence of additives, the wax crystals settle relatively quickly. For example, untreated fuels when cooled below their cloud points tend to show little wax crystal settling because the plate-like crystals interlock and cannot tumble freely in the liquid and a gel-like structure is set up but when a flow improver is added the crystals may be modified so their habit becomes less plate-like and tends to form needles of sizes in the range of tens of micrometers which can move freely in the liquid and settle relatively rapidly. This wax crystal settling can cause problems in storage tanks and vehicle systems. Concentrated wax layers may be unexpectedly drawn off, especially when the fuel level is low or the tank disturbed (e.g. when a vehicle corners), and filter blockage may occur.

If the wax crystal size can be reduced still further to below 10000 nanometers then the crystals settle relatively slowly and Wax Anti-Settling can result giving the benefits in fuel performance compared to the case of a fuel with settled wax crystals. If the wax crystal size can be reduced to below approximately 4000 nanometres then the tendency of the crystals to settle is almost eliminated within the time of fuel storage. If the crystal sizes are reduced to our optimum claim then the wax crystals remain suspended in the fuel for the many weeks required in some storage systems and the problems are effectively eliminated.

(iv) The CFPP performance which was as follows:

| Additive | CFPP Temperature | CFPP Depression |
|---|---|---|
| E | -14 | 11 |
| F | -20 | 17 |
| G | -20 | 17 |
| H | -20 | 17 |
| I | -19 | 16 |
| J | -3 | - |

(v) The average crystal size which was found to be:

| Additive | Size (Nanometers) |
|---|---|
| E | 4400 |
| F | 10400 |
| G | 2600 |
| H | 10800 |
| I | 8400 |
| J | Thin plates approximately 50000 |

**Claims**

1 Distillate fuel oil boiling in the range 120° C to 500° C which has a wax content of at least 0.3 wt.% at a temperature of 10° C below the Wax Appearance Temperature, the wax crystals at that temperature having an average particle size less than 4000 nanometres.

2 Distillate fuel according to Claim 1 in which the wax crystals have an average particle size less than 3000 nanometres.

3 Distillate fuel according to Claim 1 in which the wax crystals have an average particle size less than 2000 nanometres.

4 Distillate fuel according to Claim 1 in which the wax crystals have an average particle size less than 1500 nanometres.

5 Distillate fuel according to Claim 1 in which the wax crystals have an average particle size less than 1000 nanometres.

23·11·87

0261958

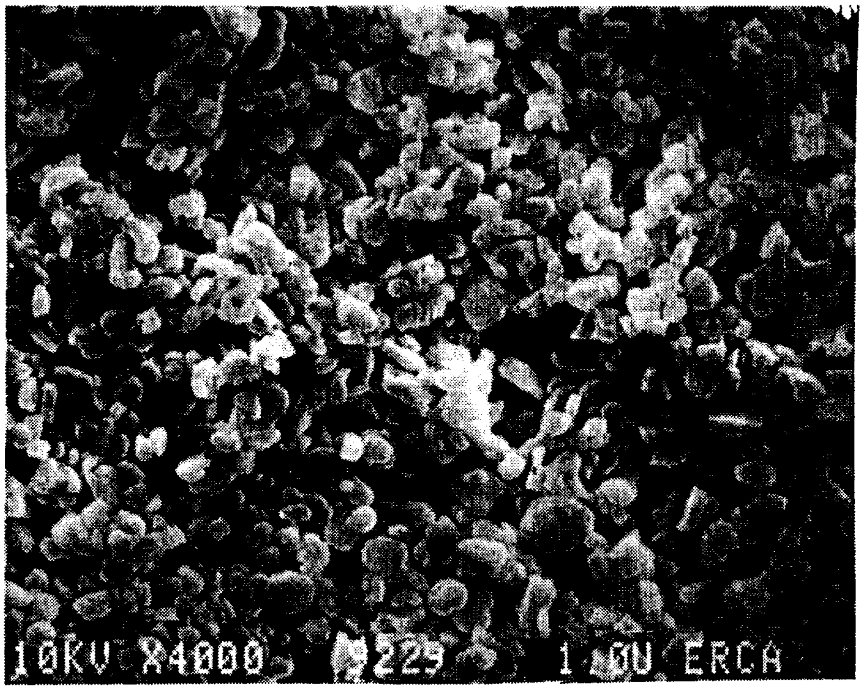


FIG.1

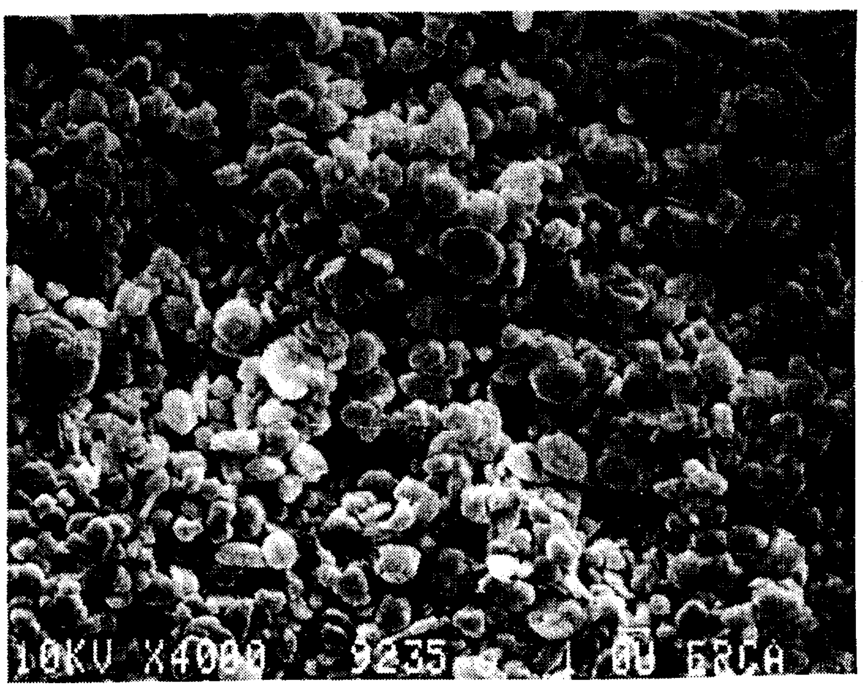


FIG.2

0261958

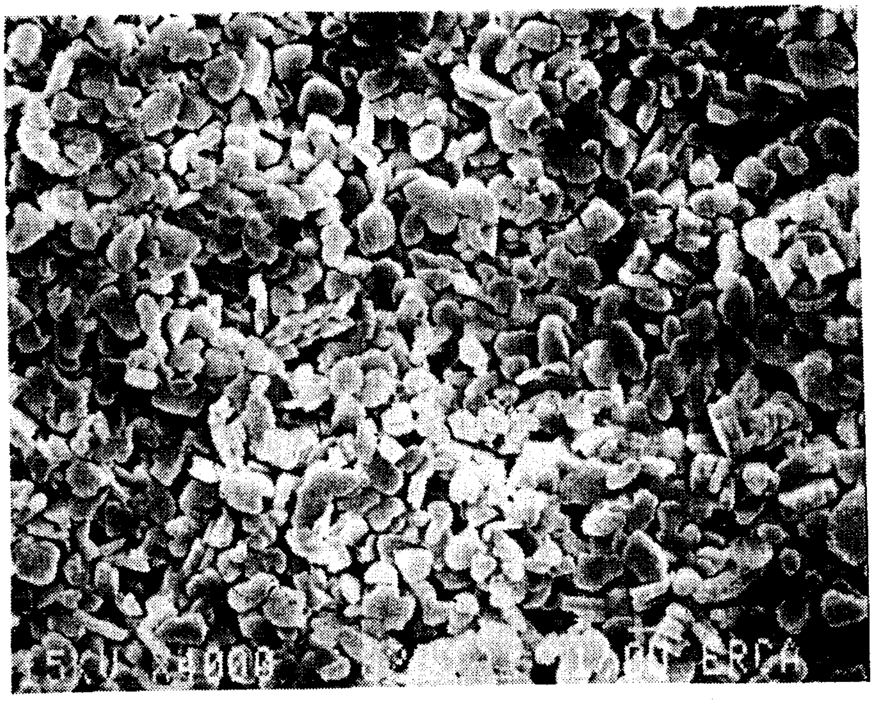


FIG.3

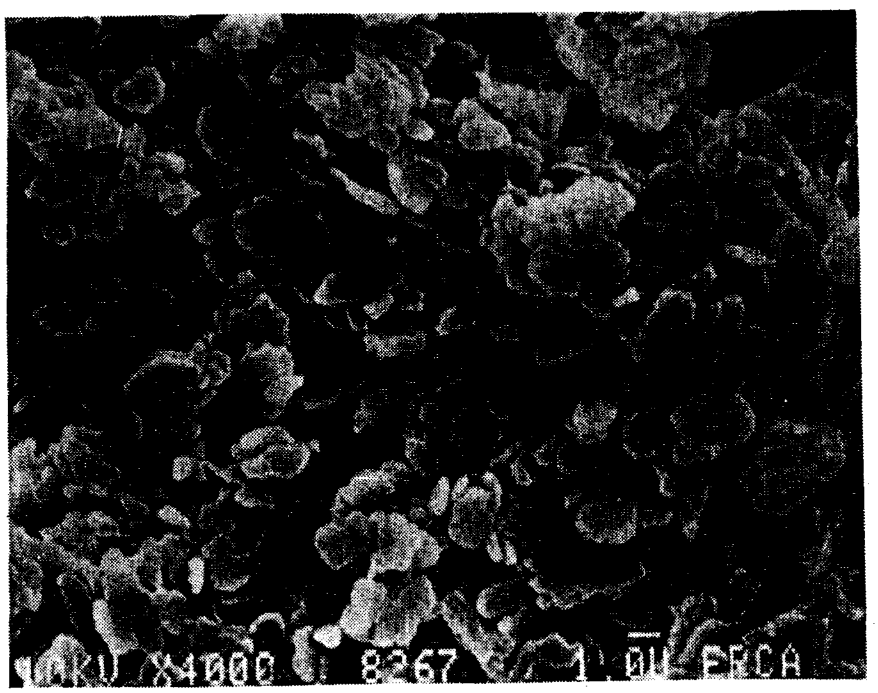


FIG.4

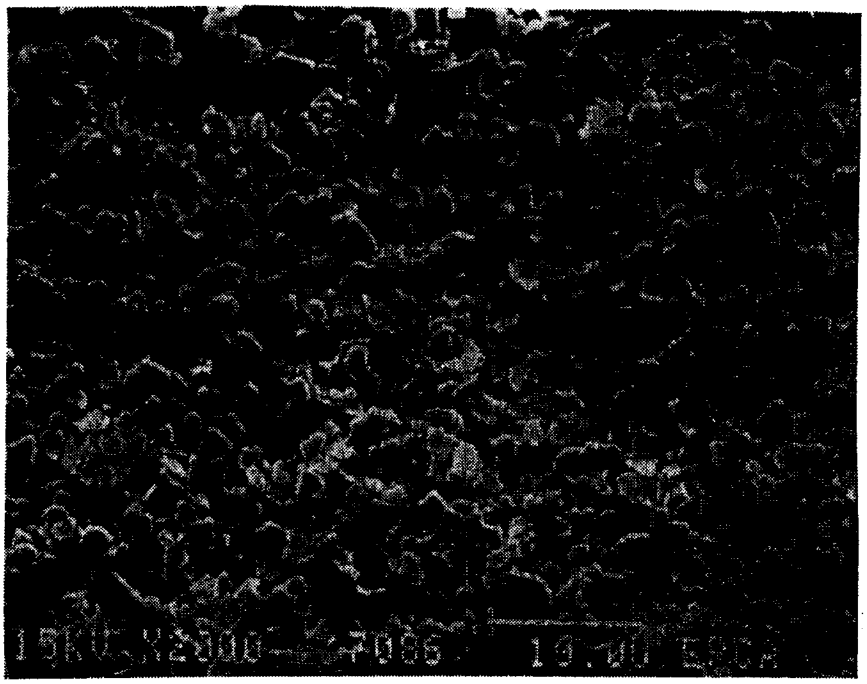


FIG.5

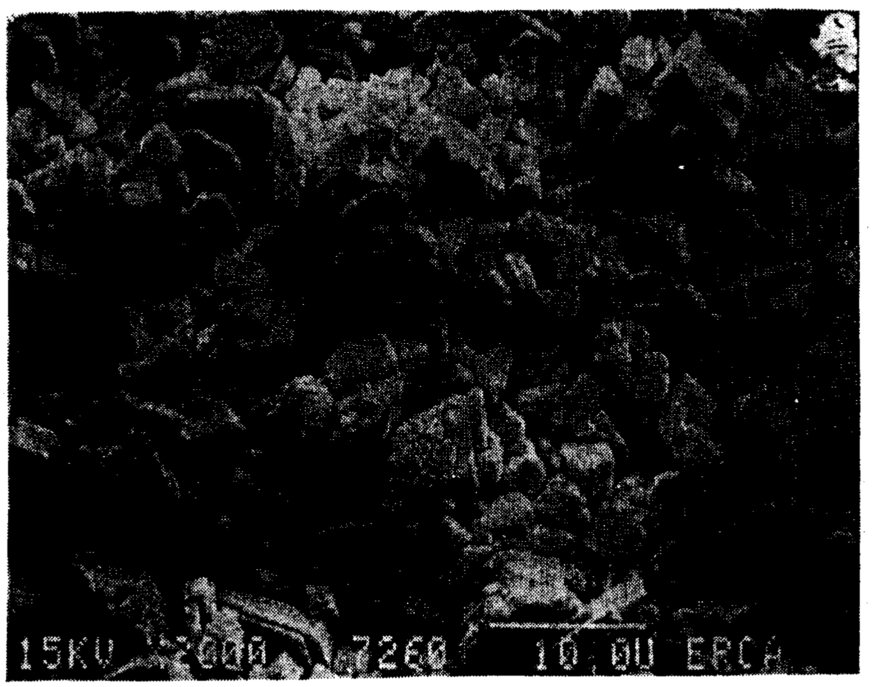


FIG.6

0261958

FIG. 7

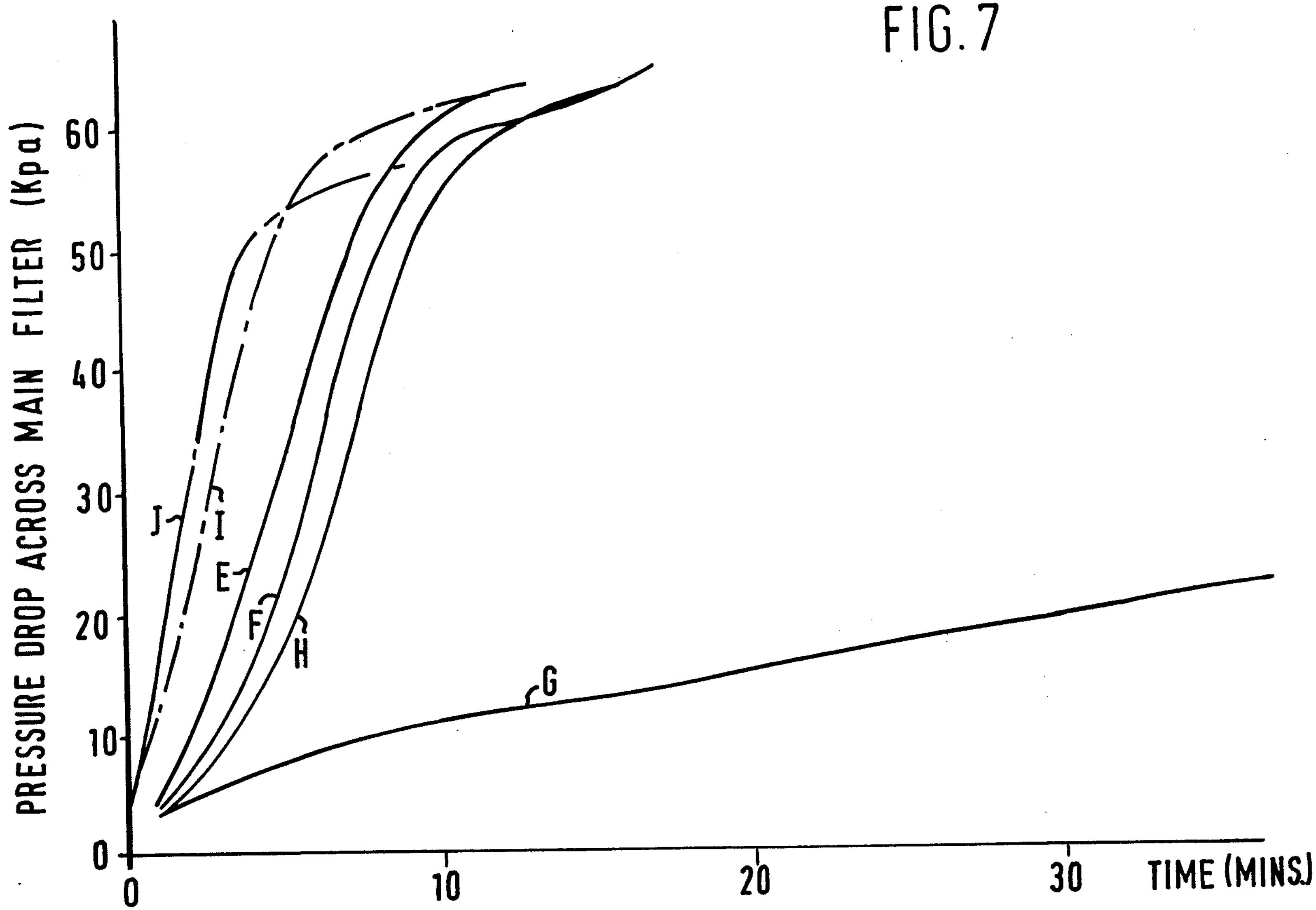

0261958

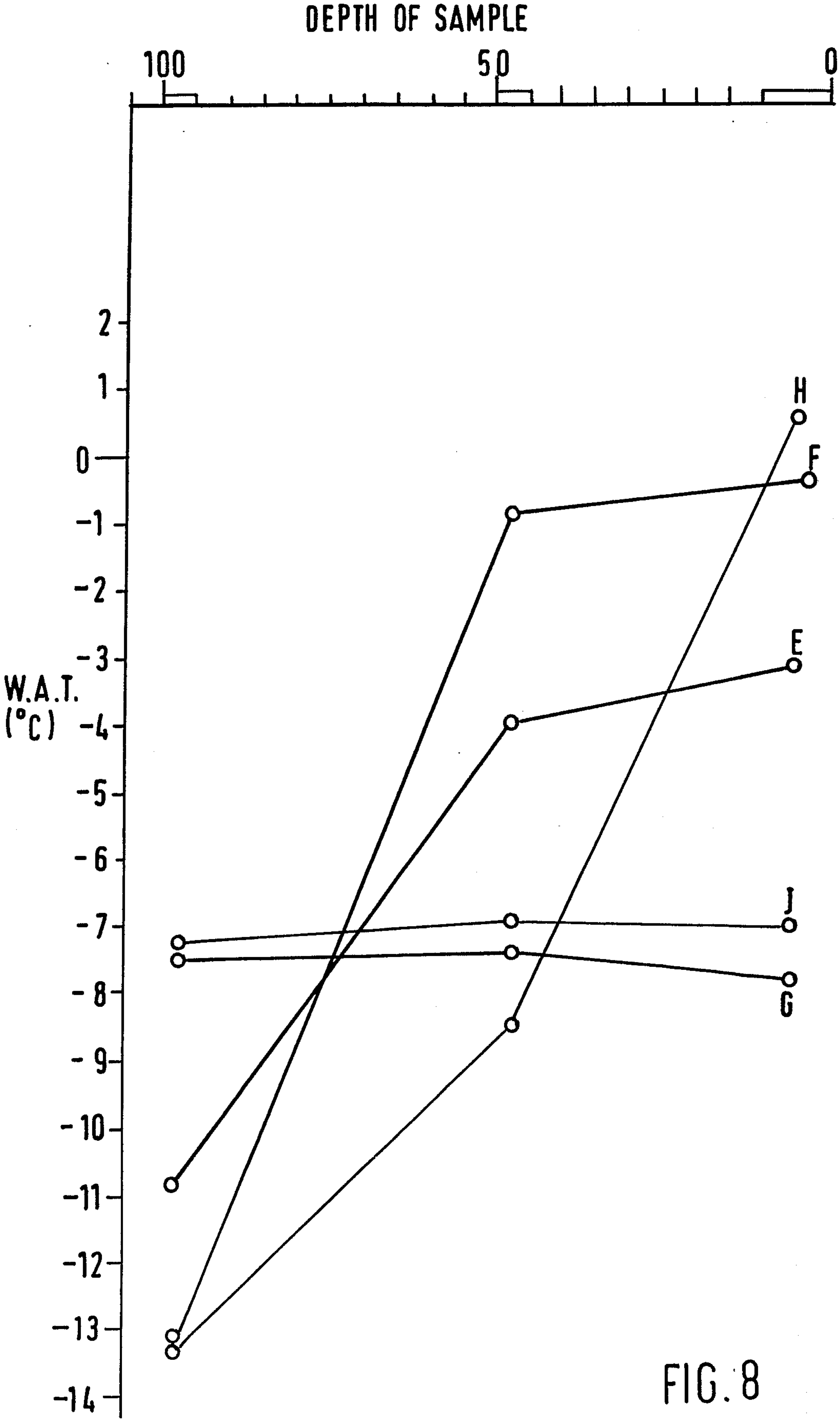


FIG. 8